# EUROPEAN PATENT SPECIFICATION

(11) **EP 4 072 832 B1**
(45) Date of publication and mention of the grant of the patent: **04.03.2026**
(21) Application number: 20898081.3
(22) Date of filing: 19.11.2020
(51) Int. Cl.: B29C 65/10, B29C 65/78, B29C 59/14, B23K 10/00, B23K 10/02, A61L 2/14, B23K 103/00, B29K 101/12, B29K 105/00

(54) **APPARATUS FOR STERILIZED WELDING**
GERÄT FÜR STERILISIERTES SCHWEISSEN
APPAREIL DE SOUDAGE STÉRILISÉ

(30) Priority: 12.12.2019 US 201962947203 P; 19.06.2020 US 202063041407 P
(43) Date of publication of application: 19.10.2022
(73) Proprietor: Saint-Gobain Performance Plastics Corporation, Solon, OH 44139 (US)
(72) Inventor: ZHANG, Jianfeng, Shrewsbury, Massachusetts 01545 (US); PYTEL, Rachel Z., Newton, Massachusetts 02461 (US); PAGLIARO, Anthony P., Lansdale, Pennsylvania 19446 (US); ZOELLNER, Clemens E., Bay City, Michigan 48706 (US); DING, Jian L., Stow, Massachusetts 01775 (US); NIXON, Thomas R., Au Gres, Michigan 48703 (US)
(74) Representative: Zimmermann & Partner Patentanwälte mbB
(86) International application number: PCT/US2020/061198
(87) International publication number: WO 2021/118780

(56) References cited:
- WO-A1-2022/094599
- WO-A2-00/62820
- JP-A- 2009 028 922
- KR-A- 20030 043 478
- KR-A- 20190 117 741
- US-A1- 2004 144 492
- US-A1- 2006 070 677

## Description

### BACKGROUND ART

Sterile connections are used to connect various profiles for the delivery and removal of fluids. Such sterile connections may be used in a variety of industries, including the medical industry and the pharmaceutical industry. Thermoplastic and thermoset elastomers are often used in such applications since these elastomers are non-toxic, flexible, thermally stable, have low chemical reactivity, and can be produced in a variety of sizes. In many instances, it is desirable to connect two different profiles to create a sterile fluid connection. Unfortunately, traditional welding apparatuses that use high temperature methods cannot effectively join thermoset elastomers, such as a silicone elastomer that cannot be melted, to another thermoset elastomer or other polymeric materials. Further, joining such materials also presents challenges in maintaining sterility at the connection. WO 00/62820 A2 discloses methods and systems for the sterile joining of components such as medical tubing.

### SUMMARY

The present disclosure relates generally to a plasma welding apparatus and methods for applying a plasma treatment to end contact surfaces of a first profile and a second profile, preferably in a sterile environment, manipulating at least one of the first profile and the second profile to force contact between the end contact surface of the first profile and the end contact surface of the second profile to permanently connect, join, or weld the end contact surfaces of the first profile and the second profile together, thereby forming a sterile connection between the first profile and a second profile.

The invention is defined by claim 1.

### BRIEF DESCRIPTION OF THE DRAWINGS

So that the manner in which the features and advantages of the embodiments are attained and can be understood in more detail, a more particular description may be had by reference to the embodiments thereof that are illustrated in the appended drawings. However, the drawings illustrate only some embodiments and therefore are not to be considered limiting in scope as there may be other equally effective embodiments.
FIG. 1 is a schematic of a plasma welding apparatus according to an embodiment of the disclosure.
FIG. 2 is a schematic diagram of a mechanical motion module according to an embodiment of the disclosure.
FIG. 3 is a schematic diagram of a mechanical motion module according to an embodiment of the disclosure.
FIG. 4 shows a first profile and a second profile in an open position for plasma welding.
FIG. 5 shows a first profile and a second profile in a closed position at a distal end of each of the first profile and the second profile for plasma welding.
FIG. 6 shows a first profile and a second profile in a closed position away from a distal end of each of the first profile and the second profile for plasma welding.
FIG. 7 is a flowchart of a method of operating a plasma welding apparatus according to an embodiment of the disclosure.
FIGS. 8A to 8G are schematic diagrams of a mechanical motion module and showing various steps of operating the mechanical motion module in a welding apparatus according to an according to an embodiment of the disclosure.
FIG. 9 is a schematic diagram of a mechanical motion module according to another embodiment of the disclosure.
FIG. 10 is a schematic diagram of a portion of a mechanical motion module according to an embodiment of the disclosure.

The use of the same reference symbols in different drawings indicates similar or identical items.

### DETAILED DESCRIPTION OF THE PREFERRED EMBODIMENT(S)

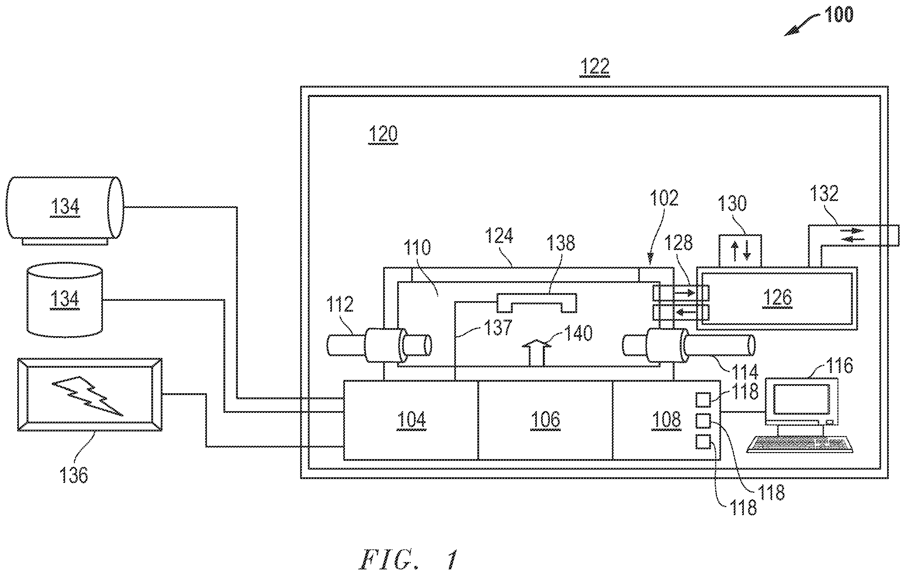

FIG. 1 shows a schematic of a plasma welding apparatus 100 according to an embodiment of the disclosure. Most generally, the plasma welding apparatus 100 may be configured to join two profiles (e.g., tubes, hoses, or other shapes or structures having a lumen through which a fluid may be carried, pumped, or otherwise transported) in a sterile environment. The joining of the two profiles may be accomplished by coincidentally welding a first end of a first profile with a second end of a second profile via a surface activation treatment. The surface activation treatment coincidentally and chemically welds the first end of the first profile to the second end of the second profile when they are placed in direct contact. Any surface activation treatment is envisioned and includes processing input energy to a surface of the first profile, the second profile, or combination thereof. In an embodiment, processing input energy is accomplished by wave irradiation, particular irradiation, or combination thereof. In an embodiment, the wave irradiation includes any wave irradiation envisioned such as radio waves, microwaves, infrared, visible light, ultraviolet, x-rays, gamma radiation, or combination thereof. In a particular embodiment, the wave irradiation includes microwaves, ultraviolet, x-rays, gamma radiation, or combination thereof. In an embodiment, the particle irradiation includes alpha radiation, beta radiation, charged ions, neutron radiation, or combination thereof. The surface activation treatment provides an effective seal between the two profiles.

The plasma welding apparatus 100 may generally comprise an operation chamber 102, a plasma generator 104, a mechanical motion module 106, and a control system 108. The operation chamber 102 may generally comprise an enclosure that defines a substantially sealed, internal environment 110 where a sterile cutting and/or plasma welding operation of a first profile 112 and a second profile 114 may occur. The plasma generator 104 may generally be configured to generate ionized particles through a plasma treatment (e.g., corona treatment, C-treatment, flame treatment, ion treatment, plasma treatment, or combinations thereof) within the operation chamber 102 that may selectively be applied to the first profile 112 and the second profile 114 to activate material on end contact surfaces of the first profile 112 and the second profile 114. The mechanical motion module 106 may generally be configured to axially or otherwise align the first profile 112 and the second profile 114 and/or force contact between the end contact surfaces of the first profile 112 and the second profile 114 to connect, join, or weld the end contact surfaces of the first profile 112 and the second profile 114 together. The control system 108 may generally comprise a user interface 116 and a plurality of sensors 118, indicators, gauges, or a combination thereof to facilitate control, monitoring, and operation of the plasma welding process.

It will be appreciated that traditional welding apparatuses that use high temperature methods cannot effectively join thermoset elastomers, such as a silicone elastomer that cannot be melted, to another thermoset elastomer or other polymeric materials. However, the plasma welding apparatus 100 is configured to connect, join, or weld a wide range of materials. In some embodiments, at least one of the first profile and the second profile may be formed from a polymeric material. However, in some embodiments, both the first profile and the second profile may be formed from a polymeric material. Accordingly, in some embodiments, the first profile and the second profile may be formed from the same polymeric material. In other embodiments, the first profile and the second profile may be formed from different polymeric materials. In some embodiments, the first profile and the second profile may be formed from a thermoplastic elastomer, a thermoset elastomer, or combination thereof. Further, in some embodiments, the thermoplastic elastomer of the first profile and/or the second profile may comprise a polystyrene, a polyester, a silicone copolymer, a silicone thermoplastic vulcanizate, a copolyester, a polyamide, a fluoropolymer, a polyolefin, a polyether-ester copolymer, a thermoplastic urethane, a polyether amide block copolymer, a polyamide copolymer, a styrene block copolymer, a polycarbonate, a thermoplastic vulcanizate, an ionomer, a polyoxymethylene (POM), an acrylonitrile butadiene styrene (ABS), an acetal, an acrylic, a polyvinyl chloride (PVC), a blend, or combination thereof. In some embodiments, the thermoset elastomer of the first profile and/or the second profile may comprise a silicone elastomer, a diene elastomer, a butyl rubber, a natural rubber, a polyurethane rubber, an ethylene propylene diene monomer rubber, an isoprene rubber, a nitrile rubber, a styrene butadiene rubber, a blend, or combination thereof.

In some embodiments, the operation chamber 102 may comprise an enclosure that defines a substantially sealed, internal environment 110 that is separated from an external environment, such as a clean room 120 and/or ambient atmosphere 122 outside the clean room 120 and/or a structure, such as a medical facility. In some embodiments, the operation chamber 102 may be configured to at least partially receive the first profile 112 and the second profile 114 to facilitate the sterile cutting and/or plasma welding operation. However, to maintain a sterile internal environment 110 within the operation chamber 102, the operation chamber 102 may be configured to maintain a fluid tight seal when the first profile 112 and the second profile 114 are received within the operation chamber 102 and partially protrudes therefrom. As such, in some embodiments, the operation chamber may function to confine a plasma or plasma treatment within the internal environment 110.

In some embodiments, the operation chamber 102 may comprise at least one transparent surface 124 through which the first profile 112 and the second profile 114 are visible. In other embodiments, the operation chamber 102 may comprise multiple transparent surfaces 124 through which the first profile 112 and the second profile 114 are visible. In yet other embodiments, the operation chamber 102 may be formed from a transparent material (e.g., acrylic, acrylic glass, plexiglass, polycarbonate, or a combination thereof) that allows observation of the first profile 112 and the second profile 114 by an operator from all angles and/or sides of the operation chamber 102. These embodiments may allow an operator to observe the plasma welding process and/or inspect the final connection between the first profile 112 and the second profile 114 for completion, quality, or a combination thereof. Further, in some embodiments, to protect the safety of an operator and/or integrity of the plasma treatment, the transparent surface(s) may at least partially block ultraviolet light from entering or exiting the enclosure.

As stated, the operation chamber 102 may maintain a substantially sterile, internal environment 110 within the operation chamber 102. In some embodiments, this may be facilitated by a two-way ventilation system 126. In some embodiments, the ventilation system 126 may exchange and/or filter air within the internal environment 110 via a first exchange system 128. In some embodiments, the ventilation system 126 may exchange and/or filter air between the clean room 120 and the internal environment 110 via a second exchange system 130. Further, in some embodiments, the ventilation system 126 may exchange and/or filter air between the ambient atmosphere 122 and the internal environment 110 via a third exchange system 132. As such, the ventilation system 126 may comprise a filter, a catalytic converter, a radiative element, or a combination thereof that treats (e.g., filters, sterilizes, reduces ozone, temperature conditions, or combinations thereof) air received from the clean room 120 via the second exchange system 130 and that enters or exits the internal environment 110 of the operation chamber 102 through the first exchange system 128. Additionally, the ventilation system 126 may also comprise a filter, a catalytic converter, a radiative element, or a combination thereof that treats air received from ambient atmosphere 122 via the third exchange system 132 and that enters or exits the internal environment 110 of the operation chamber 102 through the first exchange system 128.

The plasma generator 104 may generally comprise a gas supply 134, a power supply 136, and at least one plasma head 138 and be configured to generate ionized particles through a plasma treatment (e.g., corona treatment, C-treatment, flame treatment, ion treatment, plasma treatment, or combinations thereof) within the operation chamber 102 that may selectively be applied to the first profile 112 and the second profile 114 to activate material on end contact surfaces of the first profile 112 and the second profile 114. The gas supply 134 may be configured to provide a flow of one or more gasses, such as an inert gas, an oxygen containing gas, a nitrogen containing gas, a fluorine containing gas, or a combination thereof. In some embodiments, the gas supply 134 may comprise an atmospheric air supply, a compressor, a compressed gas cylinder, an in-house gas line, an in-house compressed gas line, a fan, a turbo, or any combination thereof to produce the flow of gas. In some embodiments, the inert gas may comprise argon, neon, helium, or any combination thereof. In other embodiments, the oxygen containing gas may comprise atmospheric air, pure oxygen, alcohol, water vapor, or a combination thereof. In yet other embodiments, the nitrogen containing gas may comprise atmospheric air, pure nitrogen, ammonia, or a combination thereof. Still, in other embodiments, the fluorine containing gas may comprises sulfur hexafluoride (SF6), trifluoromethane (CHF3), tetrafluoromethane (CF4), octafluorocyclobutane (C4F8), or a combination thereof.

The power supply 136 may generally be configured to ionize the flow of gas by imparting an electrical charge to the flow of gas to generate the plasma treatment. In some embodiments, the power supply 136 may be configured to provide an alternating current voltage of at least 110 VAC, at least 120 VAC, at least 220 VAC, or at least 240 VAC. However, in other embodiments, the power supply 136 may be configured to provide a direct current voltage of at least 6 VDC, at least 9 VDC, at least 12 VDC, at least 24 VDC, or at least 48 VDC.

The plasma treatment may be delivered to the at least one plasma head 138 disposed within the internal environment 110 of the operation chamber 102 through at least one supply line 137. The plasma head 138 may be disposed within the internal environment 110 of the operation chamber 102 and located within the operation chamber, such that the at least one plasma head 138 applies the plasma treatment to the end contact surfaces of the first profile 112 and the second profile 114. Accordingly, applying the plasma applying the plasma treatment to the end contact surfaces of the first profile 112 and the second profile 114 may comprise exposing or subjecting the end contact surfaces of the first profile 112 and the second profile 114 to the plasma treatment or delivering or directing the plasma treatment to contact or substantially envelope the end contact surfaces of the first profile 112 and the second profile 114. By applying the plasma treatment to the end contact surfaces, material at the end contact surfaces may be activated for welding the first profile 112 to the second profile 114 when they are forced into contact.

In some embodiments, the plasma generator 104 may comprise a single plasma head 138 disposed within the internal environment 110 of the operation chamber 102. However, in some embodiments, the plasma generator 104 may comprise a plurality of plasma heads 138 disposed within the internal environment 110 of the operation chamber 102. In a particular embodiment, at least one of the plurality of plasma heads 138 may be disposed adjacently to the first profile 112 within the operation chamber 102, and at least one of the plurality of plasma heads 138 may be disposed adjacently to the second profile 114 within the internal environment 110 of the operation chamber 102. Furthermore, in another particular embodiment, at least one of the plurality of plasma heads 138 may be configured to sterilize the internal environment 110 within the operation chamber 102.

The mechanical motion module 106 may generally be configured to axially or otherwise align the first profile 112 and the second profile 114 and/or force contact between the end contact surfaces of the first profile 112 and the second profile 114 to connect, join, or weld the end contact surfaces of the first profile 112 and the second profile 114 together. In some embodiments, the mechanical motion module 106 may be disposed at least partially within the operation chamber 102 or at least partially form a lower barrier or perimeter of the internal environment 110 of the operation chamber 102. In some embodiments, the mechanical motion module 106 may comprise a cutting system 140. However, in some embodiments, the cutting system 140 may be a standalone component. In some embodiments, the cutting system 140 may be disposed in the internal environment 110 of the operation chamber 102. The cutting system 140 may generally be configured to selectively cut a first tubing component to form the first profile 112 and a second tubing component to form the second profile 114. Accordingly, in some embodiments, the cutting system 140 may comprise at least one cutting device, such as at least one blade, and the at least one blade may be heated, pre-sterilized, sterilized by the plasma treatment, or a combination thereof. In some embodiments, where the at least one blade of the cutting system 140 is heated and the first tubing component 111 and the second tubing component 113 are formed from a thermoplastic elastomer, the at least one blade may be used to cut the first tubing component 111 to form the first profile 112 and cut the second tubing component 113 to form the second profile 114, which melts the ends of the profiles 112, 114, and the melted ends of the profiles 112, 114 may be joined without the use of a plasma treatment to the ends of the profiles 112, 114 but in the plasma-sterilized, internal environment 110 of the operation chamber 104. However, in some embodiments, the cutting device may comprise a laser cutting device or system.

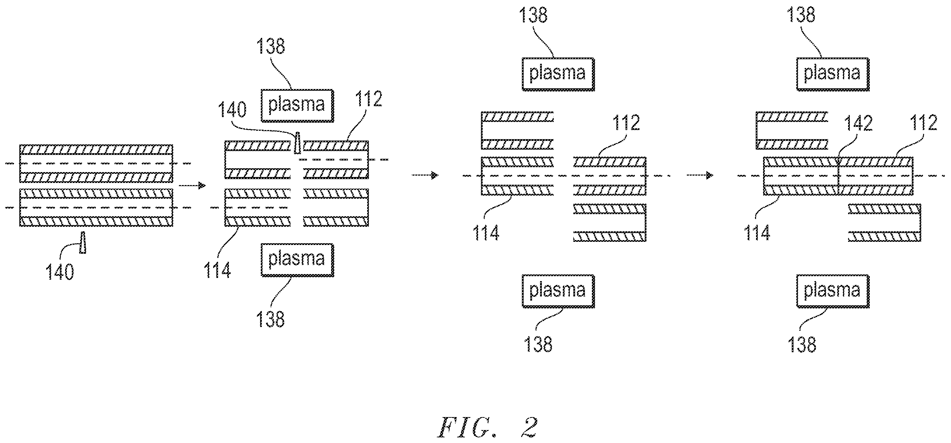

FIG. 2 shows a schematic diagram of a mechanical motion module 106 according to an embodiment of the disclosure. In the embodiment shown, the mechanical motion module 106 comprises a linear motion module. To prepare the first profile 112 and the second profile 114 for plasma welding, the cutting system 140 may be selectively operated to cut two components to form the first profile 112 and the second profile 114. In some embodiments, the plasma treatment may be applied before, during, and/or after the cutting process to activate material on the end contact surfaces of the first profile 112 and the second profile 114. The mechanical motion module 106 may be configured to axially align the end contact surfaces of the first profile 112 and the second profile 114. In some embodiments, the mechanical motion module 106 may linearly displace (substantially perpendicular to axial) at least one of the first profile 112 and the second profile 114 to axially align the end contact surfaces of the first profile 112 and the second profile 114. In other embodiments, the mechanical motion module 106 may linearly displace both the first profile 112 and the second profile 114 to axially align the end contact surfaces of the first profile 112 and the second profile 114. Additionally, once axially aligned, the mechanical motion module 106 may also be configured to force the end contact surfaces, which are activated by the plasma treatment, of the first profile 112 and the second profile 114 into contact to weld the first profile 112 and the second profile 114 at their end contact surfaces, thereby forming a joint or plasma welded connection 142. In some embodiments, the plasma treatment may continue after the first profile 112 and the second profile 114 are joined to ensure a sterile connection, thereby forming a joint or plasma welded connection 142.

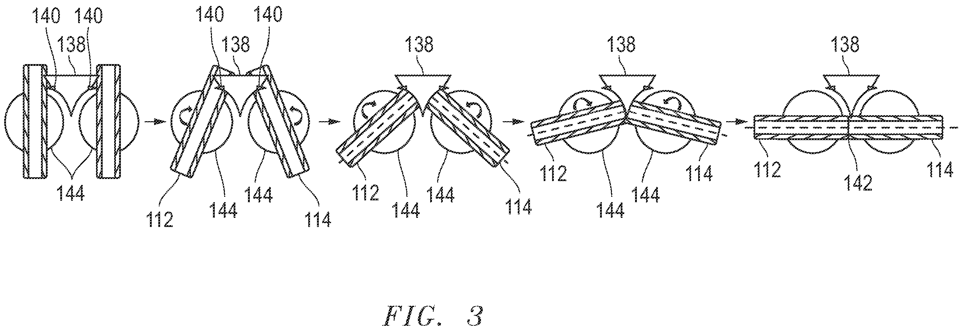

FIG. 3 shows a schematic diagram of a mechanical motion module 106 according to another embodiment of the disclosure. In the embodiment shown, the mechanical motion module 106 comprises a rotary motion module. To prepare the first profile 112 and the second profile 114 for plasma welding, two components captured by rotary motion devices 144 may be selectively rotated to bring the two components into contact with respective cutting devices to form the first profile 112 and the second profile 114. In some embodiments, the plasma treatment may be applied before, during, and/or after the cutting process to activate material on the end contact surfaces of the first profile 112 and the second profile 114. The rotary motion devices 144 of the mechanical motion module 106 may be configured to continually rotate the first profile and the second profile to axially align the end contact surfaces of the first profile 112 and the second profile 114 to axially align the end contact surfaces of the first profile 112 and the second profile 114. In other embodiments, the mechanical motion module 106 may linearly displace both the first profile 112 and the second profile 114 to axially align the end contact surfaces of the first profile 112 and the second profile 114. Additionally, once axially aligned, the rotary motion devices 144 of the mechanical motion module 106 may also be configured to force the end contact surfaces, which are activated by the plasma treatment, of the first profile 112 and the second profile 114 into contact to weld the first profile 112 and the second profile 114 at their end contact surfaces, thereby forming a joint or plasma welded connection 142. In some embodiments, the plasma treatment may continue after the first profile 112 and the second profile 114 are joined to ensure a sterile joint or plasma welded connection 142.

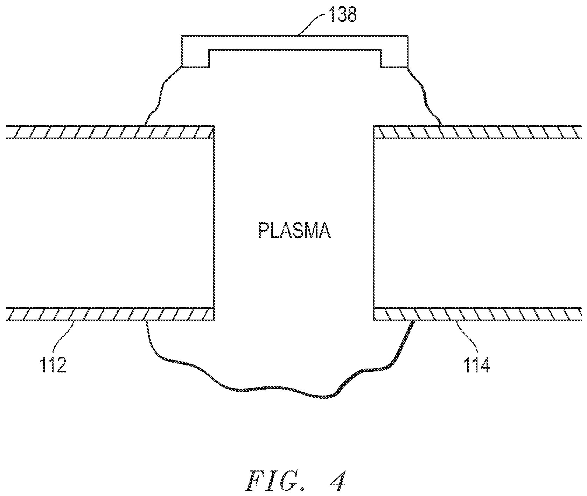

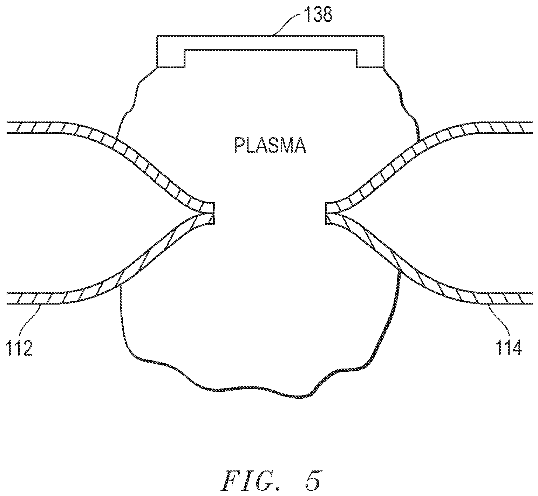

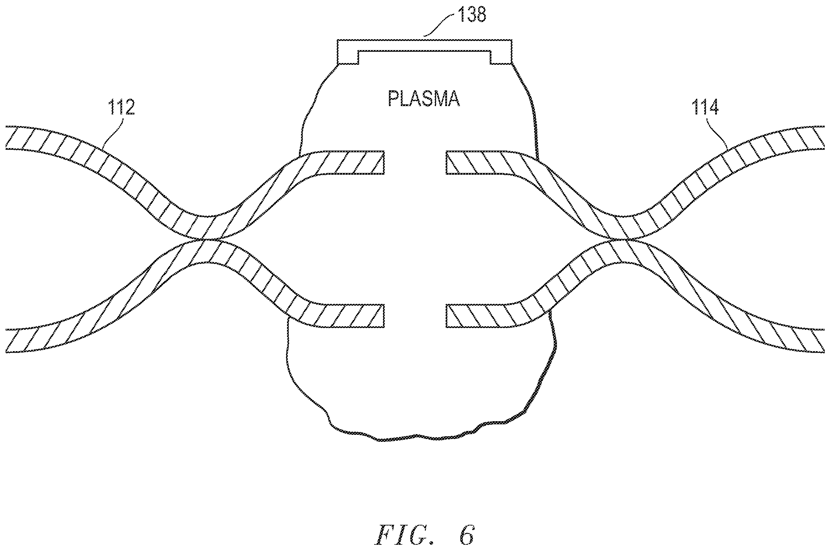

In some embodiments, the mechanical motion module 106 may be configured to manipulate one or more lumens of the first profile 112 and the second profile 114 prior to plasma welding. As such in some embodiments, the first profile 112 and the second profile 114 may remain open during the plasma treatment and/or joining the first profile 112 and the second profile 114. FIG. 4 shows the first profile 112 and the second profile 114 in an open position for plasma welding. In other embodiments, the mechanical motion module 106 may at least partially close one or more of the lumens of the first profile 112 and the second profile 114 prior to plasma welding. In some embodiments, the first profile 112 and the second profile 114 may be closed and/or sealed prior to joining the first profile 112 and the second profile 114. FIG. 5 shows the first profile 112 and the second profile 114 in a closed position at a distal end of each of the first profile 112 and the second profile 114 for plasma welding. FIG. 6 shows the first profile 112 and the second profile 114 in a closed position away from a distal end of each of the first profile 112 and the second profile 114 for plasma welding.

As stated, the plasma treatment may be applied before, during, and/or after the cutting process and/or the joining process to activate material on the end contact surfaces of the first profile 112 and the second profile 114. In some embodiments, the plasma treatment may be applied before cutting the first profile 112 and the second profile to ensure a sterile connection. In some embodiments, the plasma treatment may begin at least 1 second, at least 2 seconds, at least 3 seconds, at least 5 seconds, at least 10 seconds, at least 15 seconds at least 30 seconds, at least 45 seconds at least 60 seconds, at least 90 seconds, or at least 120 seconds before cutting the first profile 112 and the second profile 114. In some embodiments, the plasma treatment may continue after the first profile and the second profile are joined to ensure a sterile connection. In some embodiments, the plasma treatment may continue for at least 1 second, at least 2 seconds, at least 3 seconds, at least 5 seconds, at least 10 seconds, at least 15 seconds at least 30 seconds, at least 45 seconds at least 60 seconds, at least 90 seconds, or at least 120 seconds after joining the first profile 112 and the second profile 114.

Ensuring a sterile connection between the first profile 112 and the second profile 114 may be the result of pretreating the internal environment 110 of the operation chamber 104 with the plasma treatment or beginning the plasma treatment before the cutting operation of the first profile 112 and the second profile 114. Further, ensuring the sterile connection between the first profile 112 and the second profile 114 may also be the result of continuing to apply the plasma treatment after the first profile 112 and the second profile 114 are joined via plasma welding. Accordingly, the plasma treatment provides an interior environment 110 of the operation chamber 102 with a sterile environment, wherein the sterile environment is defined by a reduction in the amount of living microorganisms within the interior environment 110 of the operation chamber by a level of at least 106 after an exposure to the plasma treatment or plasma for at least 10 seconds, at least 15 seconds, at least 20 seconds, at least 25 seconds, at least 30 seconds or at least 60 seconds.

Furthermore, it will be appreciated that the plasma welding process may be performed at various temperatures within the internal environment 110 of the operation chamber 102. For example, in some embodiments, the plasma welding process may be performed at room temperature, such as that within the clean room 120. However, it will be appreciated that the plasma welding process may be performed and configured to provide a sterile connection between the first profile 112 and the second profile 114 at any temperature between about 10 degrees Celsius to 350 degrees Celsius.

The control system 108 may generally comprise a user interface 116 and a plurality of sensors 118, indicators, gauges, or a combination thereof to facilitate control, monitoring, and operation of the plasma generator 104, the mechanical motion module 106, and the plasma welding process. The user interface 116 may generally comprise a display configured to display a temperature, a gas flow rate, a gas pressure, a gas detection level, a material of the first profile and the second profile, a plasma treatment progress level, a working cycle, a total number of working cycles, or a combination thereof. In a particular embodiment, the user interface 116 may comprise a material selection input for selecting properties a material of each of the first profile 112 and the second profile 114. Furthermore, in some embodiments, the user interface 116 may comprise a warning system configured to alert an operator when an out of conformance condition exists. The user interface 116 may also be configured to automatically stop the plasma treatment when an out of conformance condition exists. Example out of conformance conditions include, but are not limited to a low temperature, a high temperature, a low pressure, a high pressure, a low gas flow rate, a high gas flow rate, a detection of a plasma byproduct, an invalid selection of a material, a leak in the operation chamber, or a combination thereof. When such conditions occur, the control system 108 may prevent opening and/or removal of the operation chamber 102. Further, the control system 108 may also prevent opening and/or removal of the operation chamber 102 during application of the plasma treatment.

The plurality of sensors 118, indicators, gauges, or combinations thereof may generally be configured to convey data (e.g., operational parameters) regarding the plasma welding operation to an operator and allow an operator to oversee the plasma welding process. In some embodiments, the plurality of sensors 118 may comprise a temperature sensor, a gas flow rate sensor, a gas pressure sensor, a gas detection sensor, a plasma byproduct sensor, a tension sensor, or a combination thereof. Further, in some embodiments, the control system 108 may be configured to log or store data transmitted from the sensors 118 related to operation of the plasma welding apparatus 100. This data may be used in troubleshooting and/or adjusting operational parameters of the components 102, 104, 106, 108 of the plasma welding apparatus 100 or the plasma welding process.

Furthermore, in some embodiments, the control system 108 may comprise at least one vision system (e.g., camera, inspection, video) configured to verify axial alignment of the end contact surfaces of the first profile 112 and the second profile 114, confirm successful joining of the first profile 112 to the second profile 114, or a combination thereof. In some embodiments, confirming successful joining of the first profile 112 to the second profile 114 may be accomplished by inspection of the welded profiles at an angle (e.g., 30 degrees, 45 degrees), where unsuccessfully joined areas appear darker than successfully joined areas. Further, in some embodiments, the control system 108 may also comprise a marking system configured to mark the first profile 112, the second profile 114, a joint or welded connection 142 formed between the first profile 112 and the second profile 114, or a combination thereof via laser marking, ink marking, or any combination thereof that allows identification, verification, troubleshooting or any combination thereof of one or more characteristics of the joint or welded connection 142 formed between the first profile 112 and the second profile 114.

The plasma welding apparatus 100 may be configured to form a sterile, plasma welded connection 142 between the first profile 112 and the second profile 114. The resulting plasma welded connection 142 may retain specific performance characteristics commensurate with an unmodified control bulk material of the first profile 112 or the second profile 114. Accordingly, in some embodiments, after the joint or plasma welded connection 142 between the first profile 112 and the second profile 114 is formed, the control system 108 may be configured to conduct a burst test, a tension test, or a combination thereof between the first profile 112 and the second profile 114 to ensure a successful plasma welded connection 142 between the first profile 112 and the second profile 114. In some embodiments, a joint or welded connection 142 formed between the first profile 112 and the second profile 114 may comprise a tensile strength of at least 10%, at least 15%, at least 25%, at least 30%, at least 35%, at least 40%, at least 45%, or at least 50% of the tensile strength of an unmodified control bulk material of the first profile 112 or the second profile 114. Furthermore, in some embodiments, a joint or welded connection 142 formed between the first profile 112 and the second profile 114 may comprise a burst pressure of at least 10%, at least 25%, at least 50%, at least 60%, at least 65%, at least 70%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, or at least 90% of the of the burst pressure of an unmodified control bulk material of the first profile or the second profile.

Still further, in some embodiments, a reinforcement may be at least partially applied about the plasma welded connection 142 formed between the first profile 112 and the second profile 114. The reinforcement may comprise an adhesive tape, a polymer tape, an overmolded polymer, a plasma-welded polymer or polymer tape, or a combination thereof.

The plasma welding apparatus 100 may generally be configured such that the operation chamber 102 and the mechanical motion module 106 are configured for mobile use and remotely coupled to the plasma generator 104 and/or the control system 108. Additionally, in some embodiments, the plasma welding apparatus 100 may be a unitary apparatus mounted to a cart or other apparatus for mobile utility, in which the entire plasma welding apparatus 100 may be moved or relocated.

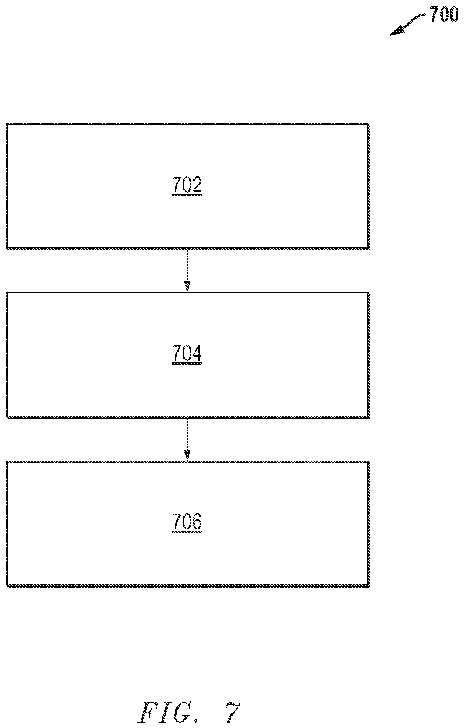

Referring to FIG. 7, a flowchart of a method 700 of operating a plasma welding apparatus 100 is shown according to an embodiment of the disclosure. The method 700 may begin at block 702 by generating a plasma treatment with a plasma generator 104. The method 700 may continue at block 704 by applying the plasma to an end contact surface of a first profile 112 and an end contact surface of a second profile 114 within an operation chamber 102. The method 700 may continue at block 706 by manipulating at least one of the first profile 112 and the second profile 114 to force contact between the end contact surface of the first profile 112 and the end contact surface of the second profile 114 to join the first profile 112 and the second profile 114.

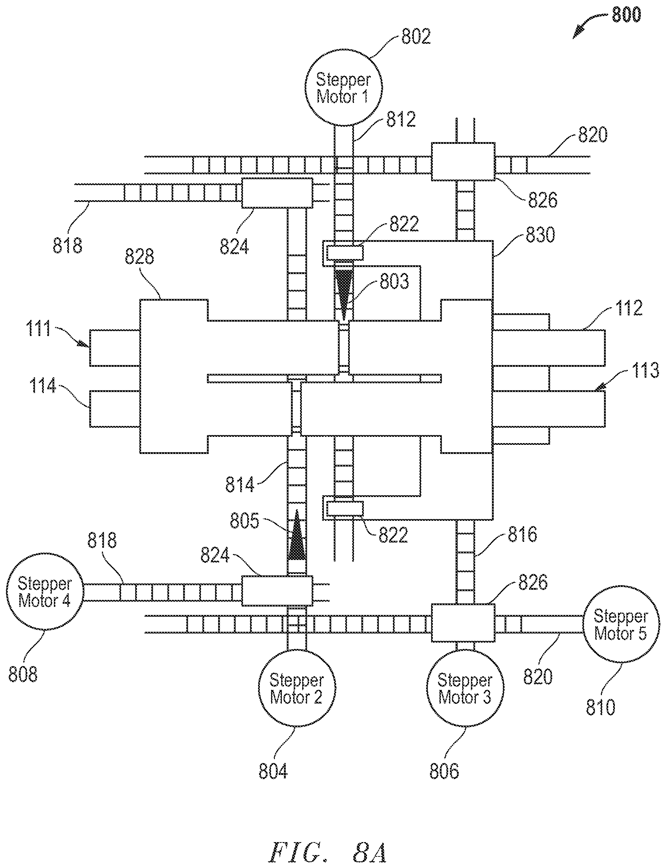

FIGS. 8A to 8G are schematic diagrams of a mechanical motion module 800 and showing various steps of operating the mechanical motion module 800 in a plasma welding apparatus 100 according to an according to an embodiment of the disclosure. The mechanical motion module 800 may generally be substantially similar to the mechanical motion module 106 and configured for operation in a plasma welding apparatus 100 in accordance with embodiments disclosed herein. As such, the mechanical motion module 800 may generally be configured to selectively cut a first tubing component 111 to form a first profile 112 and a second tubing component 113 to form a second profile 114, axially align the first profile 112 and the second profile 114 and force contact between the end contact surfaces of the first profile 112 and the second profile 114 to connect, join, or weld the end contact surfaces of the first profile 112 and the second profile 114 together when subjected to a plasma treatment. Further, it will be appreciated that in some embodiments, the mechanical motion module 800 may be disposed at least partially within the operation chamber 102 of the plasma welding apparatus 100 or at least partially form a lower barrier or perimeter of the internal environment 110 of the operation chamber 102 of the plasma welding apparatus 100.

The mechanical motion module 800 may comprise a cutting system that may be substantially similar to the cutting system 140. In some embodiments, the cutting system may be configured to selectively cut a first tubing component 111 to form the first profile 112 and a second tubing component 113 to form the second profile 114. The cutting system may generally comprise at least one cutting device 803, 805, and the cutting devices 803, 805 may comprise a blade or a laser cutting device or system. In some embodiments, the cutting system may comprise a first cutting device 803 and a second cutting device 805. More specifically, the first cutting device 803 may be configured to cut the first tubing component 111 to form the first profile 112, and the second cutting device may be configured to cut the second tubing component 113 to form the second profile 114. Cutting the first profile 112 and the second profile 114 may generally expose the end contact surfaces of the first profile 112 and the second profile 114 in accordance with embodiments disclosed herein. Further, in some embodiments, the first cutting device 803 and the second cutting device 805 may be heated, pre-sterilized, or a combination thereof, in accordance with embodiments disclosed herein. In some embodiments, where the at least one blade of the cutting system 140 is heated and the first tubing component 111 and the second tubing component 113 are formed from a thermoplastic elastomer, the at least one blade may be used to cut the first tubing component 111 to form the first profile 112 and cut the second tubing component 113 to form the second profile 114, which melts the ends of the profiles 112, 114, and the melted ends of the profiles 112, 114 may be joined without the use of a plasma treatment to the ends of the profiles 112, 114 but in the plasma-sterilized, internal environment 110 of the operation chamber 104.

The mechanical motion module 800 may comprise a plurality of actuation devices 802, 804, 806, 808, 810 configured to move various components of the mechanical motion module 800 along a plurality of rails or tracks 812, 814, 816, 818, 820 to perform the plasma welding process. In some embodiments, the actuation devices 802, 804, 806, 808, 810 may comprise an electrical stepper motor, an electro-mechanical actuator, a pneumatic actuator or cylinder, or any other suitable actuation module that may move components of the mechanical motion module 800 along the plurality of tracks 812, 814, 816, 818, 820. In some embodiments, each actuation device 802, 804, 806, 808, 810 may comprise a sensor, such as sensor 118, that is coupled to the control system 108 of the plasma welding apparatus 100. In some embodiments, each sensor may monitor and relay information associated with operation of the actuation devices 802, 804, 806, 808, 810 to the control system 118. In some embodiments, the information relayed by the sensors to the control system 108 may include directional information, positional information, and/or other operational information. In some embodiments, the plurality of tracks 812, 814, 816, 818, 820 may comprise a plurality of guides 822, 824, 826. The guides may generally be configured to control relative motion and/or maintain proper alignment of the components along the plurality of tracks 812, 814, 816, 818, 820.

The mechanical motion module 800 may also comprise a stationary tubing holder 828 and a movable tubing holder 830. The stationary tubing holder 828 may be configured to remain stationary and secure the first tubing component 111 and the second tubing component 113 in the mechanical motion module 800. In some embodiments, the stationary tubing holder 828 and the movable tubing holder 830 may extend at least partially circumferentially around each of the first tubing component 111 and the second tubing component 113 by at least 25%, at least 50%, or at least 75%. However, in some embodiments, the stationary tubing holder 828 and the movable tubing holder 830 may extend fully circumferentially about each tubing component 111, 113. In some embodiments, the stationary tubing holder 828 may comprise adjustable tubing clamps that allow for various sized tubing components 111,113 to be inserted within the stationary tubing holder 828. The movable tubing holder 830 may also be configured to secure the first tubing component 111 and the second tubing component 113 in the mechanical motion module 800. Once the first tubing component 111 and the second tubing component 113 are cut to form the first profile 112 and the second profile 114, respectively, the movable tubing holder 114 may also be configured to move both axially and transversally to axially align the end contact surfaces of the first profile 112 and the second profile 114 in order to perform the welding operation.

FIGS. 8A to 8G further depict the various steps of operating the mechanical motion module 800 in a plasma welding apparatus 100 according to an according to an embodiment of the disclosure. As shown in FIG. 8A, a first profile 112 and a second profile 114 have been inserted into the stationary tubing holder 828 and the movable tubing holder 830 and prepared to undergo a welding operation. In some embodiments, the stationary tubing holder 828 and the movable tubing holder 830 may be spaced apart a predetermined distance prior to cutting the first component 111 to form the first profile 112 and the second component 113 to form the second profile 114. In some embodiments, the predetermined distance between the stationary tubing holder 828 and the movable tubing holder 830 may be at least 1.0 mm, at least 2.0 mm, at least 3.0 mm, at least 4.0 mm, or at least 5.0 mm. In some embodiments, the predetermined distance between the stationary tubing holder 828 and the movable tubing holder 830 may be not greater than 15.0 mm, not greater than 14.0 mm, not greater than 13.0 mm, not greater than 12.0 mm, not greater than 11.0 mm, or not greater than 10.0 mm. Further, it will be appreciated that the predetermined distance between the stationary tubing holder 828 and the movable tubing holder 830 may be between any of these minimum and maximum values, such as at least 1.0 mm to not greater than 15.0 mm, or even at least 5.0 mm to not greater than 10.0 mm.

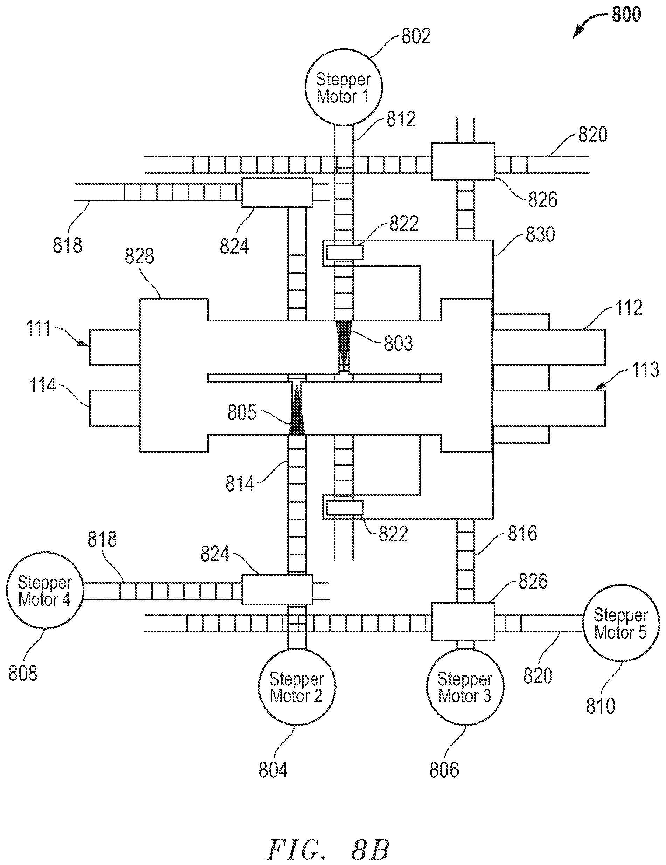

As shown in FIG. 8B, the first actuation device 802 may generally be operated to transversally move the first cutting device 803 along the first track 812 to cut the first profile 112. Guides 822 coupled to the movable tubing holder 830 may maintain proper alignment of the first cutting device 803 and the first profile 112. The second actuation device 804 may generally be operated to transversally move the second cutting device 805 along the second track 814 to cut the second profile 114. Guides 824 may maintain proper alignment of the second cutting device 805 and the second profile 114. In some embodiments, the first cutting device 803 and the second cutting device 805 may be aligned in the transversal direction. However, in other embodiments, the first cutting device 803 and the second cutting device 805 may be misaligned in the transversal direction. In some embodiments, the first cutting device 803 and the second cutting device 805 may be configured to cut the first profile 112 and the second profile 114 at an angle less than 90 degrees with respect to a length of the first profile 112 and the second profile 114. However, in other embodiments, the first cutting device 803 and the second cutting device 805 may be configured to cut the first profile 112 and the second profile 114 at a substantially orthogonally with respect to a length of the first profile 112 and the second profile 114.

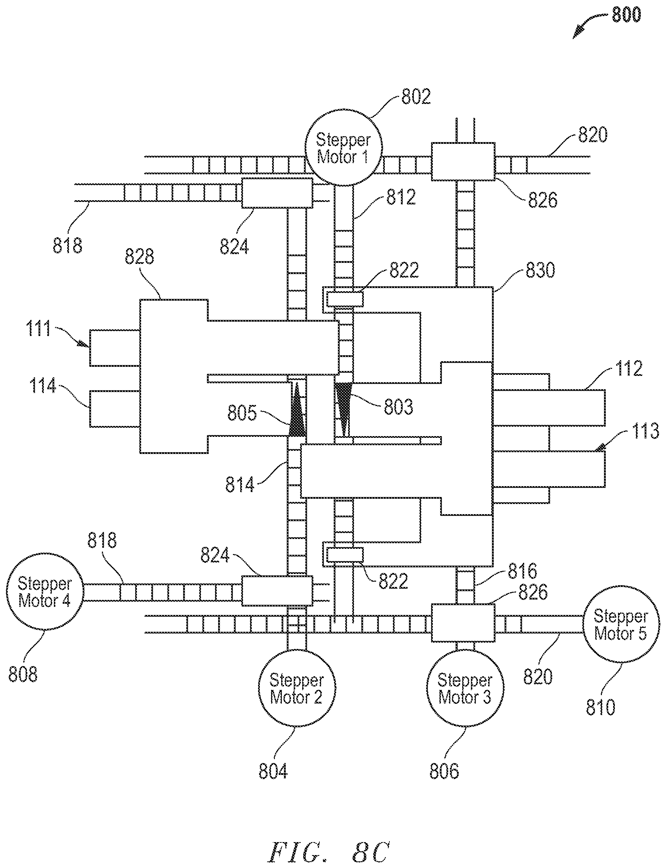

As shown in FIG. 8C, the third actuation device may generally be operated to transversally move the movable tubing holder 830 along the third track 816 to axially align the first profile 112 and the second profile 114. During movement of the movable tubing holder 830, the stationary tubing holder 828 remains stationary, so that the movable tubing holder 830 displaces transversally with respect to the stationary tubing holder 828 to axially align the first profile 112 and the second profile 114.

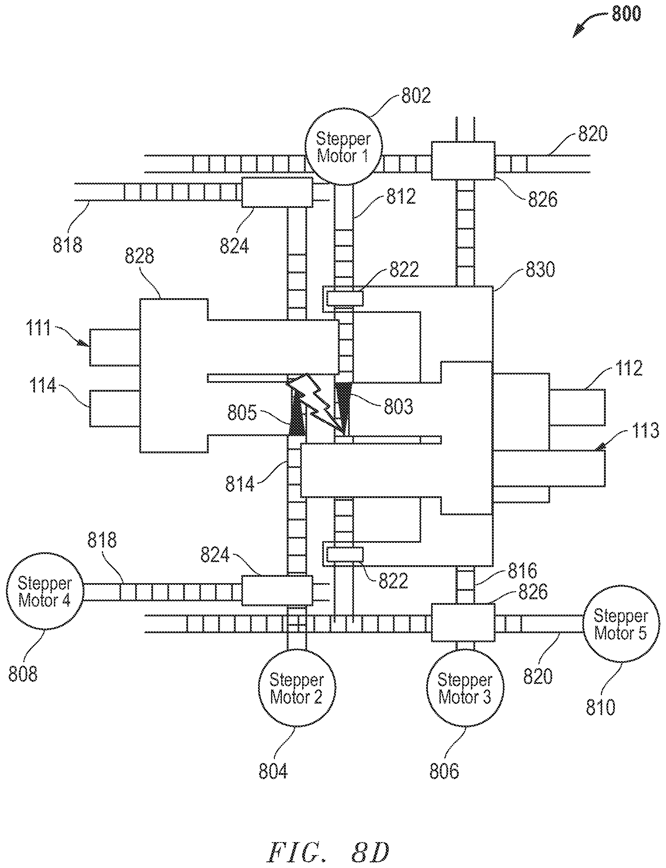

As shown in FIG. 8D, the plasma treatment may be applied to the first profile 112 and the second profile 114. More specifically, in some embodiments, the plasma treatment may be applied to the first profile 112 and the second profile 114 to activate material on the end contact surfaces of the first profile 112 and the second profile 114 in accordance with embodiments disclosed herein. Further, it will be appreciated that in some embodiments, the plasma treatment may be applied before, during, and/or after the cutting process.

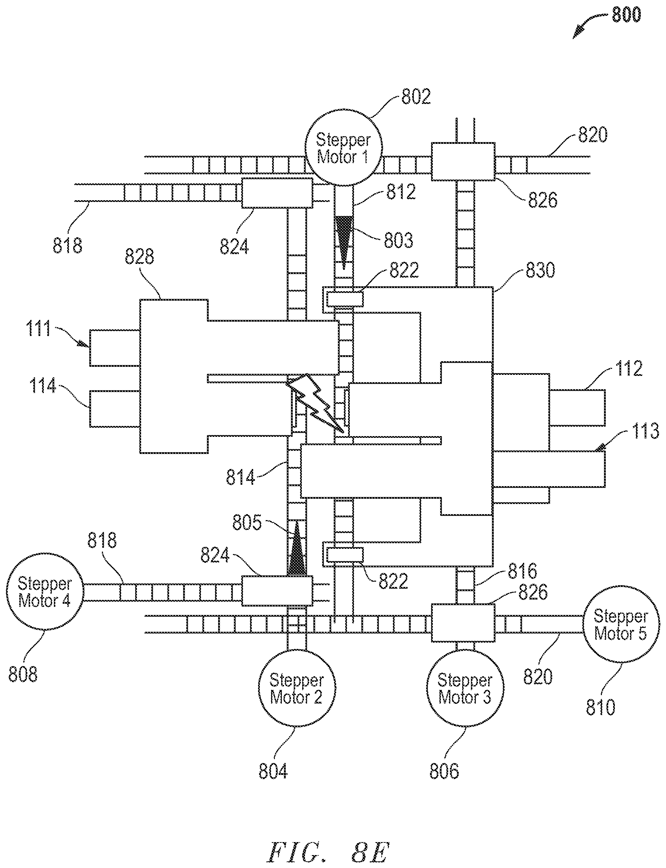

As shown in FIG. 8E, once the plasma treatment has been activated, the first actuation device 802 may generally be operated to retract the first cutting device 803 along track 812 to expose the end contact surface and/or the lumen of the first profile 112 to the plasma treatment, and the second actuation device 804 may generally be operated to retract the second cutting device 805 along track 814 to expose the end contact surface and/or the lumen of the second profile 114 to the plasma treatment. It will be appreciated that the plasma treatment may remain activated during this step in the welding process.

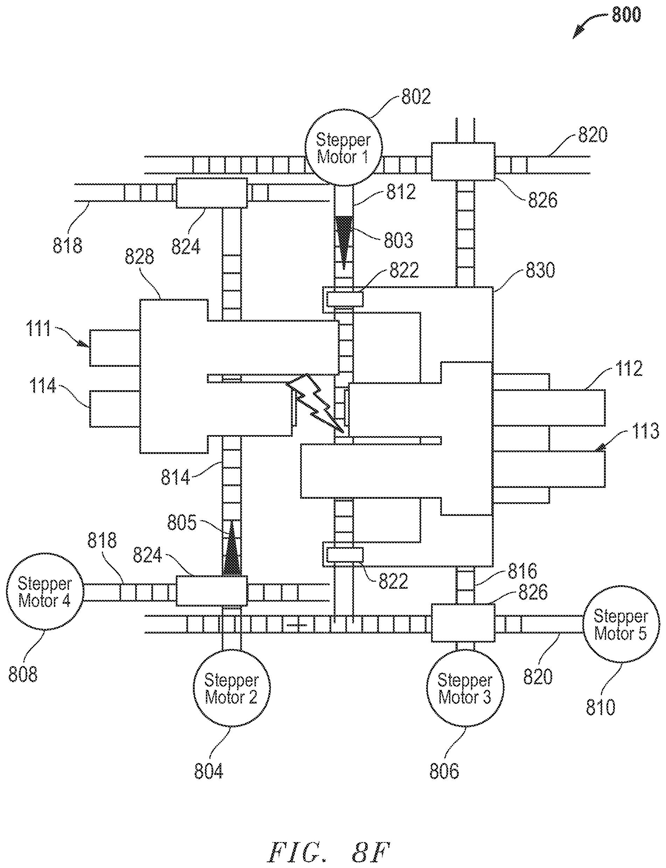

As shown in FIG. 8F, after retracting the second cutting device 805, the fourth actuation device 808 may generally be operated to axially move the second cutting device 805 along the fourth track 818. In some embodiments, the axial movement of the second cutting device 805 may provide clearance for axial movement of the movable tubing holder 830. It will be appreciated that the plasma treatment may remain activated during this step in the welding process.

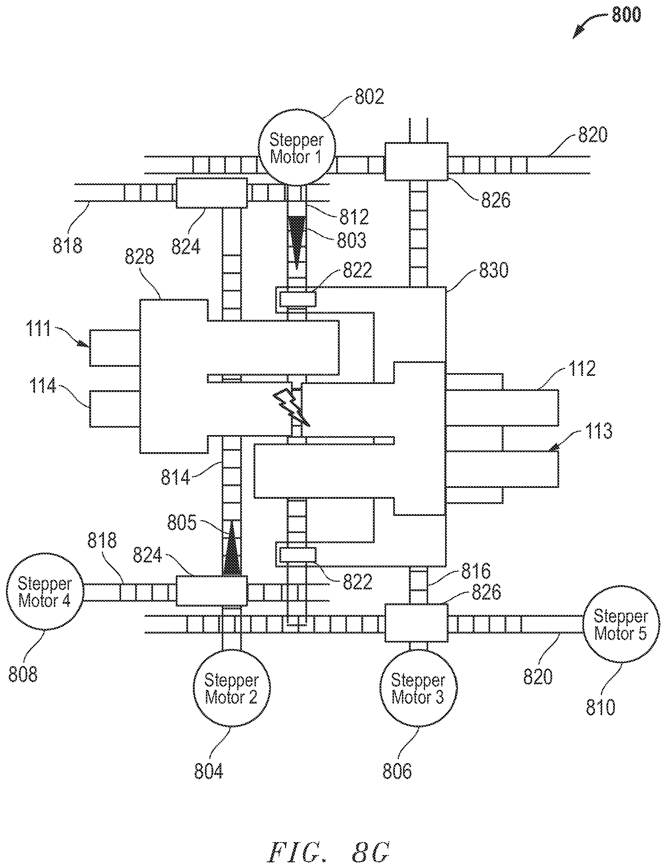

As shown in FIG. 8G, after axially moving the second cutting device 805, the fifth actuation device 810 may generally be operated to axially move the movable tubing holder 830 along the fifth track 820 to force the end contact surfaces, which are activated by the plasma treatment, of the first profile 112 and the second profile 114 into contact to weld the first profile 112 and the second profile 114 at their end contact surfaces, thereby forming a joint or plasma welded connection. In some embodiments, the plasma treatment may continue after the first profile 112 and the second profile 114 are joined to ensure a sterile connection, thereby forming a joint or plasma welded connection in accordance with embodiments disclosed herein.

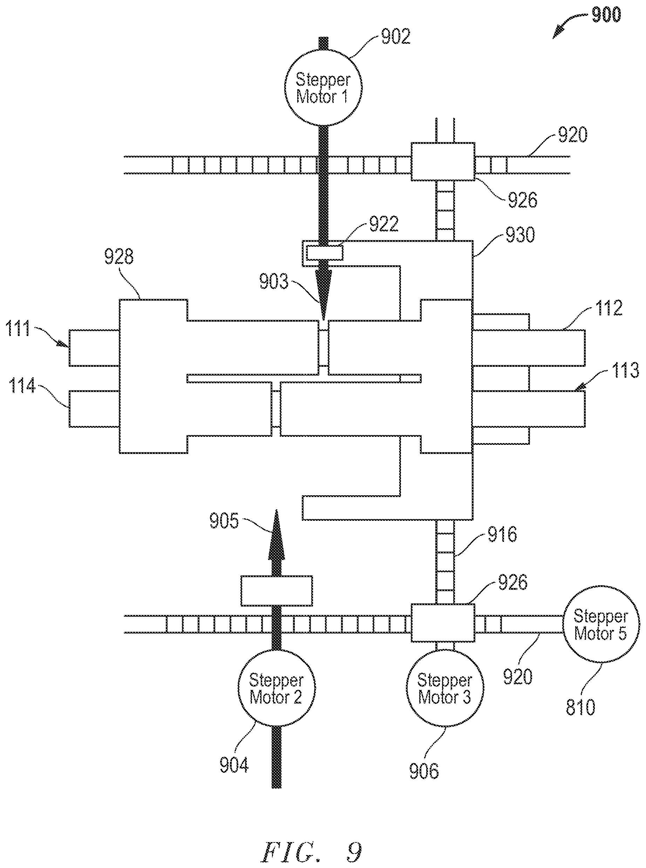

FIG. 9 shows a schematic diagram of a mechanical motion module 900 according to another embodiment of the disclosure. The mechanical motion module 900 may generally be substantially similar to the mechanical motion module 800 and configured for operation in a plasma welding apparatus 100 in accordance with embodiments disclosed herein. Similarly, the mechanical motion module 900 comprise a first cutting device 910, a second cutting device 905, a plurality of actuation devices 902, 904, 906, 910 configured to move various components of the mechanical motion module 800 along a plurality of rails or tracks 916, 918, 920, a stationary tubing holder 928, and a dynamic tubing holder 930. In some embodiments, the mechanical motion module 900 may also comprise a pair of guides 926. However, in the embodiment depicted, the mechanical motion module 900 may not include the fourth actuation device 808, the first track 812, the second track 814, the fourth track 818, and/or the guides 824. Instead, the second cutting device 905 is translated axially by the fifth actuation device 910 to provide clearance for axial movement of the movable tubing holder 930. Accordingly, in some embodiments, this may simplify operation of the welding process by eliminating the step of axially moving the second cutting device 905 (as shown in FIG. 8G) to provide clearance for axial movement of the movable tubing holder 930.

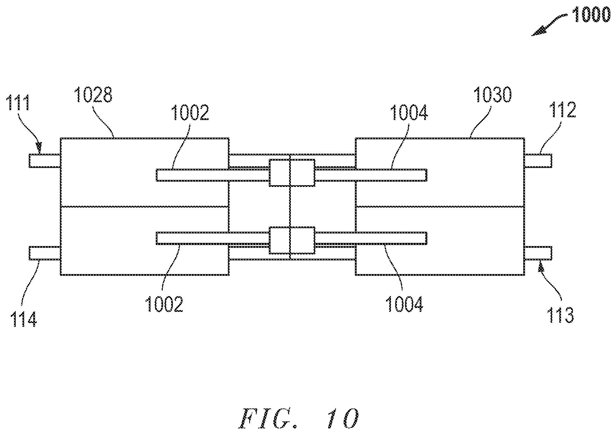

FIG. 10 is a schematic diagram of a portion of a mechanical motion module 1000 according to an embodiment of the disclosure. In some embodiments, the mechanical motion module 1000 may comprise one or more alignment rods 1002, 1004. More specifically, in the embodiment shown, the stationary tubing holder 1028 may comprise one or more alignment rods 1002, and the movable tubing holder 1030 may comprise one or more alignment rods 1004. In some embodiments, each tubing component 111, 113 may be supported by an alignment rod 1002 of the stationary tubing holder 1028 and an alignment rod of the movable tubing holder 1030 prior to cutting the tubing components 111, 113 to form the profiles 112, 114. In some embodiments, the one or more alignment rods 1002 of the stationary tubing holder 1028 and the one or more alignment rods 1004 of the movable tubing holder 1030 may be configured to abut, engage, or otherwise interact to align the stationary tubing holder 1028 and the movable tubing holder 1030. In some embodiments, the one or more alignment rods 1002 of the stationary tubing holder 1028 and the one or more alignment rods 1004 of the movable tubing holder 1030 may be configured to selectively extend and guide the first profile 112 and the second profile 114 into contact with one another such that the end contact surfaces of the first profile 112 and the second profile 114 make contact and the first profile 112 and the second profile 114 are axially aligned. In some embodiments, this may be accomplished during activation or application of the plasma treatment. Further, in some embodiments, the one or more alignment rods 1002 of the stationary tubing holder 1028 and the one or more alignment rods 1004 of the movable tubing holder 1030 may be configured to selectively extend and support the first tubing component 111 and the second component 113 prior to the cutting operation that forms the first profile 112 and the second profile 114.

In some embodiments, the mechanical motion modules 106, 800, 900, 1000 may comprise more than one of the components depicted. For example, in some embodiments, the mechanical motion module 800 may comprise two stationary tubing holder 828 and two movable tubing holders 830, such that four tubing components 111, 113 may be cut and two profiles 112, 114 may undergo a plasma welding process to form two plasma welded tubes.

Further, in some embodiments, a plasma welding apparatus 100 may comprise a plurality of one or more mechanical motion modules 106, 800, 900, 1000. In some embodiments, the plurality of mechanical motion modules 106, 800, 900, 1000 may be standalone systems or may be at least partially integrated. In some embodiments, the plurality of mechanical motion modules 106, 800, 900, 1000 may be configured to cut a plurality of tubing components 111, 113 simultaneously and weld a plurality of profiles, 112, 114.

In some embodiments, the method 700 may comprise one or more of the following: ionizing a flow of gas comprising an inert gas, an oxygen containing gas, a nitrogen containing gas, a fluorine containing gas, or a combination thereof to generate the plasma treatment; sterilizing the internal environment 110 within the operation chamber 102 by applying the plasma treatment within the operation chamber 102; cutting the first profile 112 and the second profile 114 to expose the end contact surfaces of the first profile and the second profile; applying the plasma treatment to the first profile 112 and the second profile 114 prior to cutting the first profile 112 and the second profile 114, wherein the plasma is applied to the first profile and the second profile at least 1 second, at least 2 seconds, at least 3 seconds, at least 5 seconds, at least 10 seconds, at least 15 seconds, at least 30 seconds, at least 45 seconds, at least 60 seconds, at least 90 seconds, or at least 120 seconds before cutting the first profile 112 and the second profile 114; and applying the plasma to the first profile 112 and the second profile 114 after joining the first profile 112 and the second profile 114, wherein the plasma is applied to the first profile and the second at least 1 second, at least 2 seconds, at least 3 seconds, at least 5 seconds, at least 10 seconds, at least 15 seconds, at least 30 seconds, at least 45 seconds, at least 60 seconds, at least 90 seconds, or at least 120 seconds after joining the first profile 112 and the second profile 114.

In some embodiments, the method 700 may also comprise one or more of the following: axially aligning the end contact surfaces of the first profile 112 and the second profile 114 after cutting the first profile 112 and the second profile 114; verifying the axial alignment of the end contact surfaces of the first profile 112 and the second profile 114 via a camera system; monitoring a temperature, a gas flow rate, a gas pressure, a gas detection level, a material of the first profile and the second profile, a plasma treatment progress level, a working cycle, a total number of working cycles, or a combination thereof; displaying the temperature, the gas flow rate, the gas pressure, the gas detection level, the material of the first profile 112 and the second profile 114, the plasma treatment progress level, the working cycle, the total number of working cycles, or a combination thereof on a user interface; alerting a user when an out of conformance condition exists; automatically stopping the plasma treatment when an out of conformance condition exists, wherein the out of conformance condition comprises a low temperature, a high temperature, a low pressure, a high pressure, a low gas flow rate, a high gas flow rate, a detection of a plasma byproduct, an invalid selection of a material, a leak in the operation chamber, or a combination thereof; storing data related to operation of the plasma welding apparatus 100; conducting a burst test, a tension test, or a combination thereof between the first profile and the second profile test after the first profile and the second profile are joined; selecting a material of each of the first profile 112 and the second profile 114 via the user interface; and applying a reinforcement at least partially about the coincident weld formed between the first profile and the second profile, wherein the reinforcement comprises an adhesive tape, a polymer tape, an overmolded polymer, a plasma-welded polymer, or a combination thereof.

This written description uses examples to disclose the embodiments, including the best mode, and also to enable those of ordinary skill in the art to make and use the invention. The patentable scope is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

Note that not all of the activities described above in the general description or the examples are required, that a portion of a specific activity may not be required, and that one or more further activities may be performed in addition to those described. Still further, the order in which activities are listed are not necessarily the order in which they are performed.

In the foregoing specification, the concepts have been described with reference to specific embodiments. However, one of ordinary skill in the art appreciates that various modifications and changes can be made without departing from the scope of the invention as set forth in the claims below. Accordingly, the specification and figures are to be regarded in an illustrative rather than a restrictive sense, and all such modifications are intended to be included within the scope of invention.

As used herein, the terms "comprises," "comprising," "includes," "including," "has," "having" or any other variation thereof, are intended to cover a non-exclusive inclusion. For example, a process, method, article, or apparatus that comprises a list of features is not necessarily limited only to those features but may include other features not expressly listed or inherent to such process, method, article, or apparatus. Further, unless expressly stated to the contrary, "or" refers to an inclusive-or and not to an exclusive-or. For example, a condition A or B is satisfied by any one of the following: A is true (or present) and B is false (or not present), A is false (or not present) and B is true (or present), and both A and B are true (or present).

Also, the use of "a" or "an" are employed to describe elements and components described herein. This is done merely for convenience and to give a general sense of the scope of the invention. This description should be read to include one or at least one and the singular also includes the plural unless it is obvious that it is meant otherwise.

Benefits, other advantages, and solutions to problems have been described above with regard to specific embodiments. However, the benefits, advantages, solutions to problems, and any feature(s) that may cause any benefit, advantage, or solution to occur or become more pronounced are not to be construed as a critical, required, or essential feature of any or all the claims.

After reading the specification, skilled artisans will appreciate that certain features are, for clarity, described herein in the context of separate embodiments, may also be provided in combination in a single embodiment. Conversely, various features that are, for brevity, described in the context of a single embodiment, may also be provided separately or in any subcombination. Further, references to values stated in ranges include each and every value within that range.

## Claims

1. A plasma welding apparatus (100), comprising:
an operation chamber (102); and
a plasma generator (104) having at least one plasma head (138) disposed within the operation chamber (102) and in proximity to an end contact surface of a first profile (112) within the operation chamber (102) and an end contact surface of a second profile (114) within the operation chamber (102),
wherein the plasma head (138) is configured to apply a plasma treatment to the end contact surfaces of the first profile (112) and the second profile (114) to join the first profile (112) and the second profile (114),
wherein the operation chamber (102) forms a substantially sealed environment (110), and wherein the operation chamber (102) confines the plasma treatment within the substantially sealed environment (110).

2. The plasma welding apparatus (100) of claim 1, wherein the first profile (112) and the second profile (114) are formed from a thermoplastic elastomer, a thermoset elastomer, or combination thereof.

3. The plasma welding apparatus (100) of claim 2, wherein the thermoplastic elastomer comprises a polystyrene, a polyester, a silicone copolymer, a silicone thermoplastic vulcanizate, a copolyester, a polyamide, a fluoropolymer, a polyolefin, a polyether-ester copolymer, a thermoplastic urethane, a polyether amide block copolymer, a polyamide copolymer, a styrene block copolymer, a polycarbonate, a thermoplastic vulcanizate, an ionomer, a polyoxymethylene (POM), an acrylonitrile butadiene styrene (ABS), an acetal, an acrylic, a polyvinyl chloride (PVC), a blend, or combination thereof, and wherein the thermoset elastomer comprises a silicone elastomer, a diene elastomer, a butyl rubber, a natural rubber, a polyurethane rubber, an ethylene propylene diene monomer rubber, an isoprene rubber, a nitrile rubber, a styrene butadiene rubber, a blend, or combination thereof.

4. The plasma welding apparatus (100) of claim 1, wherein the operation chamber (102) is prevented from opening or removal during application of the plasma treatment.

5. The plasma welding apparatus (100) of claim 1, wherein the plasma generator (138) comprises a gas supply and a power supply.

6. The plasma welding apparatus (100) of claim 5, wherein the gas supply comprises an atmospheric air supply, a compressor, a compressed gas cylinder, an in-house gas line, an in-house compressed gas line, a fan, a turbo, or a combination thereof, and wherein the gas supply provides a flow of gas comprising an inert gas, an oxygen containing gas, a nitrogen containing gas, a fluorine containing gas, or a combination thereof.

7. The plasma welding apparatus (100) of claim 6, wherein the inert gas comprises argon, neon, helium, or any combination thereof.

8. The plasma welding apparatus (100) of claim 6, wherein the oxygen containing gas comprises atmospheric air, pure oxygen, alcohol, water vapor, or a combination thereof, wherein the nitrogen containing gas comprises atmospheric air, pure nitrogen, ammonia, or a combination thereof, and wherein the fluorine containing gas comprises sulfur hexafluoride (SF6), trifluoromethane (CHF3), tetrafluoromethane (CF4), octafluorocyclobutane (C4F8), or a combination thereof.

9. The plasma welding apparatus (100) of claim 6, wherein the power supply ionizes a flow of gas from the gas supply to generate the plasma treatment.

10. The plasma welding apparatus (100) of claim 1, further comprising: a plurality of plasma heads (138) disposed within the operation chamber (102), wherein at least one of the plurality of plasma heads (138) is configured to sterilize the environment within the operation chamber (102).

11. The plasma welding apparatus (100) of claim 1, further comprising: a cutting device (803, 805) disposed in the operation chamber (102) and configured to cut the first profile (112) and the second profile (114).

12. The plasma welding apparatus (100) of claim 1, further comprising: a mechanical motion module (106) configured to axially align the end contact surfaces of the first profile (112) and the second profile (114).

13. The plasma welding apparatus (100) of claim 1, further comprising: a control system (108) configured to automatically stop the plasma treatment when an out of conformance condition exists.

## Patentansprüche

1. Plasmaschweißvorrichtung (100), umfassend:
eine Betriebskammer (102); und
einen Plasmagenerator (104) mit mindestens einem Plasmakopf (138), der innerhalb der Betriebskammer (102) und in der Nähe einer Endkontaktfläche eines ersten Profils (112) innerhalb der Betriebskammer (102) und einer Endkontaktfläche eines zweiten Profils (114) innerhalb der Betriebskammer (102) angeordnet ist,
wobei der Plasmakopf (138) konfiguriert ist, um eine Plasmabehandlung auf die Endkontaktflächen des ersten Profils (112) und des zweiten Profils (114) anzuwenden, um das erste Profil (112) und das zweite Profil (114) zu verbinden,
wobei die Betriebskammer (102) eine im Wesentlichen abgedichtete Umgebung (110) bildet, und wobei die Betriebskammer (102) die Plasmabehandlung innerhalb der im Wesentlichen abgedichteten Umgebung (110) begrenzt.

2. Plasmaschweißvorrichtung (100) nach Anspruch 1, wobei das erste Profil (112) und das zweite Profil (114) aus einem thermoplastischen Elastomer, einem duroplastischen Elastomer oder einer Kombination davon gebildet sind.

3. Plasmaschweißvorrichtung (100) nach Anspruch 2, wobei das thermoplastische Elastomer ein Polystyrol, ein Polyester, ein Silikon-Copolymer, ein thermoplastisches Silikon-Vulkanisat, ein Copolyester, ein Polyamid, ein Fluorpolymer, ein Polyolefin, ein Polyether-Ester-Copolymer, ein thermoplastisches Urethan, ein Polyetheramid-Blockcopolymer, ein Polyamid-Copolymer, ein Styrol-Blockcopolymer, ein Polycarbonat, ein thermoplastisches Vulkanisat, ein lonomer, ein Polyoxymethylen (POM), ein Acrylnitril-Butadien-Styrol (ABS), ein Acetal, ein Acryl, ein Polyvinylchlorid (PVC), eine Mischung oder eine Kombination davon umfasst, und wobei das duroplastische Elastomer ein Silikon-Elastomer, ein Dien-Elastomer, einen Butylkautschuk, einen Naturkautschuk, einen Polyurethan-Kautschuk, einen Ethylen-Propylen-Dien-Kautschuk, einen Isopren-Kautschuk, einen Nitril-Kautschuk, einen Styrol-Butadien-Kautschuk, eine Mischung oder eine Kombination davon umfasst.

4. Plasmaschweißvorrichtung (100) nach Anspruch 1, wobei die Betriebskammer (102) während der Anwendung der Plasmabehandlung an einem Öffnen oder Entfernen gehindert wird.

5. Plasmaschweißvorrichtung (100) nach Anspruch 1, wobei der Plasmagenerator (138) eine Gasversorgung und eine Energieversorgung umfasst.

6. Plasmaschweißvorrichtung (100) nach Anspruch 5, wobei die Gasversorgung eine Atmosphärenluftversorgung, einen Kompressor, eine Druckgasflasche, eine hausinterne Gasleitung, eine hausinterne Druckgasleitung, einen Lüfter, einen Turbo oder eine Kombination davon umfasst, und wobei die Gasversorgung einen Gasstrom bereitstellt, der ein Inertgas, ein sauerstoffhaltiges Gas, ein stickstoffhaltiges Gas, ein fluorhaltiges Gas oder eine Kombination davon umfasst.

7. Plasmaschweißvorrichtung (100) nach Anspruch 6, wobei das Inertgas Argon, Neon, Helium oder jeder Kombination davon umfasst.

8. Plasmaschweißvorrichtung (100) nach Anspruch 6, wobei das sauerstoffhaltige Gas Atmosphärenluft, reinen Sauerstoff, Alkohol, Wasserdampf oder eine Kombination davon umfasst, wobei das stickstoffhaltige Gas Atmosphärenluft, reinen Stickstoff, Ammoniak oder eine Kombination davon umfasst, und wobei das fluorhaltige Gas Schwefelhexafluorid (SF6), Trifluormethan (CHF3), Tetrafluormethan (CF4), Octafluorcyclobutan (C4F8) oder eine Kombination davon umfasst.

9. Plasmaschweißvorrichtung (100) nach Anspruch 6, wobei die Energieversorgung einen Gasstrom aus der Gasversorgung ionisiert, um die Plasmabehandlung zu erzeugen.

10. Plasmaschweißvorrichtung (100) nach Anspruch 1, ferner umfassend: eine Vielzahl von Plasmaköpfen (138), die innerhalb der Betriebskammer (102) angeordnet sind, wobei mindestens einer der Vielzahl von Plasmaköpfen (138) konfiguriert ist, um die Umgebung innerhalb der Betriebskammer (102) zu sterilisieren.

11. Plasmaschweißvorrichtung (100) nach Anspruch 1, ferner umfassend: eine Schneidvorrichtung (803, 805), die in der Betriebskammer (102) angeordnet und konfiguriert ist, um das erste Profil (112) und das zweite Profil (114) zu schneiden.

12. Plasmaschweißvorrichtung (100) nach Anspruch 1, ferner umfassend: ein mechanisches Bewegungsmodul (106), das konfiguriert ist, um die Endkontaktflächen des ersten Profils (112) und des zweiten Profils (114) axial auszurichten.

13. Plasmaschweißvorrichtung (100) nach Anspruch 1, ferner umfassend: ein Steuersystem (108), das konfiguriert ist, um die Plasmabehandlung automatisch zu stoppen, wenn eine Nichtkonformitätsbedingung vorliegt.

## Revendications

1. Appareil de soudage plasma (100), comprenant :
une chambre de fonctionnement (102) ; et
un générateur de plasma (104) ayant au moins une tête de plasma (138) disposée à l'intérieur de la chambre d'opération (102) et à proximité d'une surface de contact d'extrémité d'un premier profil (112) à l'intérieur de la chambre d'opération (102) et d'une surface de contact d'extrémité d'un second profil (114) à l'intérieur de la chambre d'opération (102),
dans lequel la tête de plasma (138) est conçue pour appliquer un traitement au plasma aux surfaces de contact d'extrémité du premier profil (112) et du second profil (114) pour joindre le premier profil (112) et le second profil (114),
dans lequel la chambre de fonctionnement (102) forme un environnement sensiblement scellé (110), et dans lequel la chambre de fonctionnement (102) confine le traitement au plasma dans l'environnement sensiblement scellé (110).

2. Appareil de soudage au plasma (100) selon la revendication 1, dans lequel le premier profil (112) et le second profil (114) sont formés d'un élastomère thermoplastique, d'un élastomère thermodurcissable ou d'une combinaison de ceux-ci.

3. Appareil de soudage plasma (100) selon la revendication 2, dans lequel l'élastomère thermoplastique comprend un polystyrène, un polyester, un copolymère de silicone, un vulcanisat thermoplastique de silicone, un copolyester, un polyamide, un fluoropolymère, une polyoléfine, un copolymère d'ester de polyéther, un uréthane thermoplastique, un copolymère séquencé de polyétheramide, un polyamide copolymère, un copolymère séquencé de styrène, un polycarbonate, un vulcanisat thermoplastique, un ionomère, un polyoxyméthylène (POM), un styrène acrylonitrile butadiène (ABS), un acétal, un acrylique, un chlorure de polyvinyle (PVC), un mélange, ou une combinaison de ceux-ci, et dans lequel l'élastomère thermodurci comprend un élastomère de silicone, un élastomère diénique, un butyle caoutchouc, un caoutchouc naturel, un caoutchouc polyuréthane, un caoutchouc éthylène propylène diène un caoutchouc monomère, un caoutchouc isoprène, un caoutchouc nitrile, un caoutchouc styrène caoutchouc de butadiène, un mélange, ou une combinaison de ceux-ci.

4. Appareil de soudage au plasma (100) selon la revendication 1, dans lequel la chambre de fonctionnement (102) est empêchée de s'ouvrir ou de se retirer pendant l'application du traitement au plasma.

5. Appareil de soudage en plasma (100) selon la revendication 1, dans lequel le générateur de plasma (138) comprend une alimentation en gaz et une alimentation électrique.

6. Appareil de soudage au plasma (100) selon la revendication 5, dans lequel l'alimentation en gaz comprend une alimentation en air atmosphérique, un compresseur, une bouteille de gaz comprimé, une conduite de gaz interne, une conduite de gaz comprimé interne, un ventilateur, un turbo, ou une combinaison de ceux-ci, et dans lequel l'alimentation en gaz fournit un flux de gaz comprenant un gaz inerte, un gaz contenant de l'oxygène, un gaz contenant de l'azote, un gaz contenant du fluor ou une combinaison de ceux-ci.

7. Appareil de soudage au plasma (100) selon la revendication 6, dans lequel le gaz inerte comprend de l'argon, du néon, de l'hélium ou une combinaison quelconque de ceux-ci.

8. Appareil de soudage au plasma (100) selon la revendication 6, dans lequel le gaz contenant de l'oxygène comprend de l'air atmosphérique, de l'oxygène pur, de l'alcool, de la vapeur d'eau ou une combinaison de ceux-ci, dans lequel le gaz contenant de l'azote comprend de l'air atmosphérique, de l'azote pur, de l'ammoniac ou une combinaison de ceux-ci, et dans lequel le gaz contenant du fluor comprend de l'hexafluorure de soufre (SF6), du trifluorométhane (CHF3), du tétrafluorométhane (CF4), de l'octafluorocyclobutane (C4F8), ou une combinaison de celui-ci.

9. Appareil de soudage au plasma (100) selon la revendication 6, dans lequel l'alimentation électrique ionise un flux de gaz provenant de l'alimentation en gaz pour générer le traitement au plasma.

10. Appareil de soudage plasma (100) selon la revendication 1, comprenant en outre : une pluralité de têtes plasma (138) disposées dans la chambre d'opération (102), dans lequel au moins l'une de la pluralité de têtes plasma (138) est conçue pour stériliser l'environnement à l'intérieur de la chambre d'opération (102).

11. Appareil de soudage plasma (100) selon la revendication 1, comprenant en outre : un dispositif de coupe (803, 805) disposé dans la chambre d'opération (102) et conçu pour couper le premier profil (112) et le second profil (114).

12. Appareil de soudage plasma (100) selon la revendication 1, comprenant en outre : un module de déplacement mécanique (106) conçu pour aligner axialement les surfaces de contact d'extrémité du premier profil (112) et du second profil (114).

13. Appareil de soudage au plasma (100) selon la revendication 1, comprenant en outre : un système de commande (108) configuré pour arrêter automatiquement le traitement au plasma lorsqu'une condition de non-conformité existe.
